# EUROPÄISCHE PATENTSCHRIFT

(11) **EP 3 638 158 B1**
(45) Veröffentlichungstag und Bekanntmachung des Hinweises auf die Patenterteilung: **30.07.2025**
(21) Anmeldenummer: 18729948.2
(22) Anmeldetag: 06.06.2018
(51) Int. Cl.: A61F 5/01, A61F 2/50, A61F 2/64, A61F 2/68

(54) **GELENKEINRICHTUNG**
JOINT DEVICE
DISPOSITIF ARTICULAIRE

(30) Priorität: 12.06.2017 DE 102017112911
(43) Veröffentlichungstag der Anmeldung: 22.04.2020
(73) Patentinhaber: Otto Bock Healthcare Products GmbH, 1110 Wien (AT)
(72) Erfinder: AUBERGER, Roland, 1140 Wien (AT); BREUER-RUESCH, Christian, 1230 Wien (AT); KREPPER, Sebastian, 2282 Markgrafneusiedl (AT)
(74) Vertreter: Gramm, Lins & Partner Patent- und Rechtsanwälte PartGmbB
(86) Internationale Anmeldenummer: PCT/EP2018/064918
(87) Internationale Veröffentlichungsnummer: WO 2018/228892

(56) Entgegenhaltungen:
- EP-B1- 2 254 532
- WO-A1-01/21114
- WO-A1-2011/123928
- DE-A1- 102008 024 748
- DE-B3- 102006 012 716
- US-A- 6 039 707
- US-A1- 2015 230 962

## Beschreibung

Die Erfindung betrifft eine Gelenkeinrichtung für eine Prothese oder Orthese oder einer Prothese oder Orthese mit einem Oberteil, einem Unterteil, mit einem Gelenk, das eine Gelenkachse aufweist, um die das Oberteil relativ zu dem Unterteil verschwenkbar gelagert ist und einem Aktuator, der ausgebildet ist, eine Verschwenkung des Oberteils relativ zu dem Unterteil zu beeinflussen, wobei der Aktuator an dem Oberteil an eine Oberteilbefestigungsstelle und an dem Unterteil an eine Unterteilbefestigungsstelle gelagert ist.

Gelenkeinrichtungen können in Orthesen oder Prothesen eingesetzt werden. Orthesen werden unter anderem dazu eingesetzt, um die Funktion der noch vorhandenen Extremität zu unterstützen oder aufrechtzuerhalten. Dazu wird die Orthese an der noch vorhandenen Extremität festgelegt. Bei einer Orthese der unteren Extremität, beispielsweise einer das Kniegelenk übergreifenden Orthese, werden an dem Oberschenkel und an dem Unterschenkel Schienen angelegt, die über ein Orthesenkniegelenk miteinander verbunden sind. Bei einer Knöchelorthese wird der Fuß auf einem Fußteil fixiert, das über ein Orthesenknöchelgelenk mit einer Unterschenkelschiene verbunden ist. Gleiches gilt für eine Hüftgelenksorthese oder für eine Orthese, die mehr als zwei natürliche Gelenke überbrückt. Darüber hinaus können Orthesen auch an oberen Extremitäten eingesetzt werden.

Fehlende Gliedmaßen werden durch Prothesen ersetzt. Fehlen natürliche Gelenke, beispielsweise ein Kniegelenk, wird das fehlende natürliche Kniegelenk durch ein Prothesenkniegelenk ersetzt. Das Oberteil des Prothesenkniegelenkes wird an einen Oberschenkelstumpf über einen Oberschenkelschaft festgelegt. An dem Oberschenkelteil ist das Unterteil des Prothesenkniegelenkes schwenkbar befestigt. An dem Unterschenkelteil sind ein Unterschenkelrohr und ein Prothesenfuß, gegebenenfalls mit einem Prothesenknöchelgelenk angeordnet.

Um die Bewegungen der einzelnen Komponenten, sowohl bei Orthesen als auch bei Prothesen, zu beeinflussen, beispielsweise um eine Verschwenkbewegung zu unterstützen oder zu behindern, können Aktuatoren eingesetzt werden. Eine Bewegungsunterstützung erfolgt über die Zufuhr von Energie aus einem Energiespeicher, beispielweise über einen Federmechanismus, oder aber über einen motorischen Antrieb, der die Energie aus einem Energiespeicher zur Speicherung in der Regel elektrischer Energie vorsieht. Zur Behinderung einer Bewegung, beispielsweise zum Dämpfen einer Flexions- oder Extensionsbewegung oder zum Abbremsen werden Dämpfer eingesetzt, beispielsweise Hydraulikdämpfer oder Pneumatikdämpfer. Es können Anschlagelemente in der jeweiligen Gelenkeinrichtung eingesetzt werden, um bei Erreichen einer Maximalstellung, sei es Flexion oder Extension, einen weichen Anschlag zu gewährleisten.

Eine Gelenkeinrichtung für untere Extremitäten ist beispielsweise aus der DE 10 2015 113 799 A1 bekannt.

Die DE 10 2006 012 716 B3 betrifft eine Gelenkeinrichtung für Orthesen oder Prothesen, mit einem Oberteil und einem um eine Schwenkachse drehbar daran gelagerten Unterteil. Eine Bremseinrichtung bremst eine Schwenkbewegung des Unterteils relativ zu dem Oberteil oder blockiert diese Schwenkbewegung, wobei eine Spiralfeder zwischen zwei Anlaufflächen angeordnet ist, die um eine Verdrehachse parallel zu der Schwenkachse verspannbar gelagert ist. Bei einer Verspannung in Richtung auf die Anbauflächen tritt die Spiralfeder mit diesen in Kontakt.

Die WO 2011/123928 A1 betrifft eine Lastverteilungsvorrichtung oder Orthese, die muskuläre und knöcherne Belastungen an einem Gelenk auf benachbarte Körperbereiche überträgt. Dazu sind ein proximales und ein distales Abstützelement gelenkig miteinander verbunden und weisen Befestigungseinrichtungen zum Festlegen an dem jeweiligen Körperbereich auf. Ein Kompensationsgelenk ist koaxial zu der Drehachse des Gelenkes zwischen den beiden Abstützelementen angeordnet.

Die US 6 039 707 A betrifft eine Beckenabstützung als Gehhilfevorrichtung mit einem Oberkörperabstützteil, einem Oberschenkelteil und einem Gelenk im Bereich des Hüftgelenks. Eine Feder ist mit dem Gelenk gekoppelt, um eine Beinschwäche auszugleichen. Über eine Vorspanneinrichtung kann die Federvorspannung eingestellt werden, um eine Anpassung an unterschiedliche Situationen und den jeweiligen Patienten vornehmen zu können.

Die EP 2 254 532 B1 betrifft eine Vorrichtung zur konservativen oder postoperativen Behandlung eines Hüftgelenkes. Zwei Hüftstützen werden beidseitig des Beckens angelegt, an diesen sind Oberschenkelstützen gelenkig befestigt. Das Gelenk weist zumindest einen Stift oder Gewindestift auf, der in einen Abduktions-Einstellnocken eingreift. Eine Vorrichtung zur Einstellung der Abduktion hinsichtlich einer Verzögerung oder Blockierung vorgesehen. Der Bewegungsbereich des Gelenkes kann eingestellt werden. Das Gelenk ist längsverschieblich an Streben an der Beckenabstützung bzw. einer Oberschenkelabstützung angeordnet.

Die WO 01/21114 A1 betrifft ein Scharniergelenk mit einer Winkeleinstellbarkeit mit einem ersten Scharnierelement und einem zweiten Scharnierelement, die durch einen ersten zylindrischen Bolzen miteinander um dessen Längsachse schwenkbar gekoppelt sind. Der erste Bolzen ist an dem ersten Scharnierelement befestigt weist eine Reihe von Zähnen auf, die radial nach außen zeigen. Ein zweiter Bolzen ist dem zweiten Scharnierelement befestigt. Der zweite Bolzen ist drehbar um eine zweite Achse senkrecht zu der ersten Achse des ersten Bolzens verschwenkbar und weist ein Gewinde auf, das mit den Zähnen des ersten Bolzens kämmt. Eine Drehung des zweiten Bolzens führt zu einer Drehung des ersten Bolzens um dessen Achse. Ein solches Scharnier kann an einer Orthese zwischen einem Gelenk ist einer Befestigungseinrichtung an einer Gliedmaße angeordnet sein.

Die DE 10 2008 024 748 A1 und die US 2015/0230962 A1, die als nächstliegender Stand der Technik angesehen werden kann, betreffen jeweils eine Knieorthese mit einer Oberschenkelschiene und einer Unterschenkelschiene, die über eine Gelenkeinrichtung miteinander verbunden sind. Ein Hydraulikaktuator ist zwischen der Oberschenkelschiene und der Unterschenkelschiene angeordnet und dort an Lagerungspunkten festgelegt, die als Kardangelenke oder Kugelgelenke beschrieben sind.

Insbesondere in der Orthetik, aber auch in der Prothetik, besteht bislang die Notwendigkeit, dass vorhandene Passteile parallel zu erhaltenen Körperteilen angebracht werden müssen, damit Schwenkachsen miteinander fluchten. Insbesondere bei seitlich neben einer Extremität angeordneten Aktuatoren wie Dämpfern oder Antrieben wird dadurch ein hohes Bauvolumen verursacht, da die Komponenten nicht der Körperkontur folgend angeordnet werden können.

Aufgabe der vorliegenden Erfindung ist es, eine Gelenkeinrichtung bereitzustellen, die leicht an den jeweiligen Nutzer anpassbar ist und ein möglichst geringes Volumen benötigt.

Erfindungsgemäß wird diese Aufgabe durch eine Gelenkeinrichtung mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Die Gelenkeinrichtung für eine Prothese oder Orthese oder einer Prothese oder Orthese mit einem Oberteil, einem Unterteil, mit einem Gelenk, das eine Gelenkachse aufweist, um die das Oberteil relativ zu dem Unterteil verschwenkbar gelagert ist, und einem Aktuator, der ausgebildet ist, eine Verschwenkung des Oberteils relativ zu dem Unterteil zu beeinflussen, wobei der Aktuator an dem Oberteil an eine Oberteilbefestigungsstelle und an dem Unterteil an einer Unterteilbefestigungsstelle gelagert ist, sieht vor, dass zwischen der Oberteilbefestigungsstelle und der Unterteilbefestigungsstelle zumindest zwei Gelenke angeordnet sind, dass die Gelenke eine Verschwenkung des Aktuators zu der Oberteilbefestigungsstelle und der Unterteilbefestigungsstelle ermöglichen und jeweils zumindest eine Schwenkachse ausbilden, von denen zumindest eine nicht parallel zu der Gelenkachse orientiert ist. Der Aktuator muss nicht unmittelbar an dem Oberteil und dem Unterteil befestigt sein, er kann über Zwischenstücke, Befestigungsteile, Verlängerungen oder weitere Bauteile an der jeweiligen Unterteilbefestigungsstelle oder Oberteilbefestigungsstelle gelagert sein. Dadurch, dass zwischen der Oberteilbefestigungsstelle und der Unterteilbefestigungsstelle zumindest zwei Gelenke angeordnet sind, besteht die Möglichkeit, dass der Aktuator auch bei einer nicht geradlinigen Ausrichtung der Oberteilbefestigungsstelle zu der Unterteilbefestigungsstelle und somit bei einer geneigten oder verdrehten Orientierung des Aktuators zu der Gelenkachse eine möglichst körpernahe bzw. nahe an dem Oberteil oder Unterteil gelegene Befestigung des Aktuators erfolgen kann. Somit ist es möglich, dass die Anlenkung des Aktuators und die mechanische Struktur, die die Kräfte des Aktuators aufnimmt und im Fall einer Orthese an die Gliedmaßen überträgt, so ausgeführt werden kann, dass eine Anpassung an die Körperkontur möglich ist, ohne dass eine manuelle Bearbeitung zur Anformung der Passteile erforderlich ist. Gleiches gilt für Prothesen, bei denen die mechanische Struktur in der Regel aus einem Schaft als Oberteil und einem gelenkig daran gelagerten Unterteil besteht und über den Aktuator Dämpfungskräfte oder Antriebskräfte auf das Oberteil und Unterteil übertragen werden. Da üblicherweise Orthesenkomponenten in verschiedenen Formen und Winkeln als Standardteile angeboten werden, müssen diese durch mechanische Bearbeitung wie Verformungen oder Kürzungen an die Kontur der jeweiligen Extremität, beispielsweise des Beines, angepasst werden. Ähnliches gilt bei Prothesen, deren Schäfte häufig als Einzelteilanfertigungen maßgefertigt werden oder als Testprothesenschäfte individuell an die Körperkontur anpassbar sind. Um einen Aktuator an der Prothese oder Orthese festzulegen, ist häufig eine mechanische Nachbearbeitung notwendig oder aber Distanzstücke sind vorzusehen, damit die Komponenten, die einer Extensions- oder Flexionsbewegung folgen, üblicherweise in einer Ebene bewegt werden, die lotrecht auf der Schwenkachse steht. Mit der Anordnung oder Ausbildung zumindest zweier Gelenke, die eine Verschwenkung des Aktuators zu der Oberteilbefestigungsstelle und der Unterteilbefestigungsstelle ermöglichen jeweils eine Schwenkachse ausbilden, von denen zumindest eine nicht parallel zu der Gelenkachse orientiert ist, ist es möglich, den Winkel des Aktuators zur optimalen Anpassung an die Körperkontur einzustellen.

In der Erfindung ist es vorgesehen, dass der Aktuator an einer Halterung befestigt ist, die zwischen der Gelenkachse und der Oberteilbefestigungsstelle oder der Unterteilbefestigungsstelle angeordnet ist. Dabei ist in der Halterung zumindest eine der Schwenkachsen ausgebildet. Bei der Halterung handelt es sich somit um ein Zwischenstück, das an dem Unterteil oder dem Oberteil festgelegt ist oder festlegbar ist.

An der Halterung ist ein Teil des Aktuators befestigt, beispielsweise bei einem Linearaktuator das Gehäuse oder die sich in das Gehäuse und aus dem Gehäuse herausbewegende Betätigungsstange, beispielsweise eine Kolbenstange oder ein motorisch ausfahrbare Komponente. Das jeweils andere Ende des Aktuators kann dann an der anderen Komponente der Gelenkeinrichtung, also an dem Unterteil oder an dem Oberteil befestigt sein und Kräfte des Aktuators auf die jeweilige Komponente übertragen. Durch die Ausgestaltung einer Halterung zur Befestigung zwischen dem Aktuator und dem Oberteil oder dem Unterteil ist es möglich, ein standardisierbares, vorfertigbares Modul bereitzustellen, das an dem Oberteil oder dem Unterteil an der jeweiligen Befestigungsstelle festgelegt wird und eine Kopplungsstelle oder eine Lagerstelle mit dem Aktuator auszubilden. Dadurch können kostengünstige Standardbauteile verwendet werden, um Standardkomponenten an die individuelle Körperkontur des jeweiligen Nutzers anpassen zu können.

Eine Weiterbildung der Erfindung sieht vor, dass die erste Schwenkachse orthogonal zu der Gelenkachse orientiert ist, so dass eine Verkippung senkrecht zu der Gelenkachse möglich ist. Dadurch kann eine Verkippung aus der lotrecht zur Schwenkachse orientierten Ebene leicht erfolgen.

In einer Weiterbildung der Erfindung ist vorgesehen, dass die erste Schwenkachse orthogonal zu der zweiten Schwenkachse orientiert ist. In einer Weiterbildung ist vorgesehen, dass beide Schwenkachsen nicht parallel zueinander und auch nicht parallel zu der Gelenkachse orientiert sind, wodurch sich eine nahezu beliebige Einstellbarkeit der Orientierung des Aktuators zu der Gelenkachse und der jeweiligen Befestigungsstelle ermöglichen lässt.

Die beiden Schwenkachsen liegen bevorzugt in einer gemeinsamen Ebene und schneiden sich bevorzugt in einem Punkt. Der Schnittpunkt der beiden Schwenkachsen muss nicht ein Punkt auf der Gelenkachse sein, liegt aber bevorzugt auf der Gelenkachse, so dass alle drei Achsen sich in einem gemeinsamen Punkt schneiden.

Bevorzugt ist der Aktuator als Linearaktuator ausgebildet und bewirkt entweder aktiv eine Verschwenkung des Oberteils relativ zu dem Unterteil oder dämpft oder bremst eine Verschwenkbewegung des Oberteils relativ zu dem Unterteil.

Der Aktuator kann als elektrischer, pneumatischer oder hydraulischer Antrieb bei einer aktiven Unterstützung der jeweiligen Bewegung oder als hydraulischer oder pneumatischer Dämpfer oder als elektrische, pneumatische, mechanische oder magnetische Bremse ausgebildet sein, wenn der Aktuator eine bremsende oder dämpfende Wirkung haben soll.

In einer Weiterbildung der Erfindung ist es vorgesehen, dass zumindest eine der Schwenkachsen in einer Schwenkebene liegt, die orthogonal zu der Gelenkachse orientiert ist. Dadurch kann eine Verdrehung des Aktuators um die Schwenkachse erfolgen, ohne dass die Bewegung um die Gelenkachse beeinträchtigt wird.

Die Halterung kann eine Halteplatte aufweisen, die drehfest an dem Oberteil oder dem Unterteil festgelegt ist, um Kräfte und Momente auf das Oberteil oder das Unterteil wirksam übertragen zu können. Darüber hinaus wird durch die drehfeste Festlegung der Halterung an dem Oberteil oder dem Unterteil eine ausreichend stabile Führung des Aktuators an dem Oberteil oder dem Unterteil erreicht.

Wenn der Aktuator als Linearaktuator ausgebildet ist, ist bevorzugt eine Lagerstelle beabstandet zu der Gelenkachse an der Halterung vorgesehen, an der der Aktuator entweder mit dem Gehäuse oder der ausfahrbaren Stange an der Halterung gelagert ist. Dadurch ist es möglich, ein Moment um die Gelenkachse zu bewirken oder aufzunehmen, um eine Verschwenkung des Oberteils relativ zu dem Oberteil zu bewirken oder zu beeinflussen.

In einer Weiterbildung der Erfindung ist der Aktuator an einem Rahmen festgelegt, der eine Befestigungseinrichtung oder mehrere Befestigungseinrichtungen zur reversiblen Anordnung an dem Oberteil und dem Unterteil aufweist. Über diesen Rahmen ist es möglich, eine standardisierte Schnittstelle beispielsweise bei einer darauf befestigten Steuerung, Sensoren oder anderen Komponenten herzustellen. Darüber hinaus lässt sich über den Rahmen eine Schnittstelle für den Aktuator herstellen, so dass unterschiedliche Oberteile oder Unterteile mit Befestigungsanschlüssen für die Befestigungseinrichtungen vorab ausgestattet werden können, so dass unterschiedliche Komponenten an dem Rahmen getestet werden können. Insbesondere für Testorthesen oder Testprothesen ist eine solche Ausgestaltung mit einem Rahmen vorteilhaft, da die jeweiligen Komponenten einfach an unterschiedlichen Oberteilen, Unterteilen oder aber auch unterschiedliche Komponenten an den jeweiligen Rahmen befestigt werden können, so dass eine Vielzahl von Kombinationen ohne mechanische Veränderungen an den Oberteilen, Unterteilen oder Passstücken oder der Einsatz einer Vielzahl von Distanzelementen notwendig wäre.

In einer Weiterbildung der Erfindung ist vorgesehen, dass die Halterung schwenkbar an dem Rahmen befestigt ist und die Gelenkachse und/oder eine Schwenkachse zwischen dem Rahmen und der Halterung ausgebildet ist. Die Halterung und der Rahmen bilden somit das eigentliche, Kräfte und Momente übertragene Gelenk zwischen dem Oberteil und dem Unterteil. Die Kombination aus Halterung und Rahmen kann unabhängig von dem Oberteil und dem Unterteil ausgestaltet sein. Das Oberteil oder Unterteil einer Prothese sind beispielweise ein Prothesenschaft und ein sich distal daran anschließendes weiteres Prothesenbauteil wie beispielsweise Unterschenkelrohr, Prothesenfuß oder bei oberen Extremitäten ein Unterarmrohr, während bei einer Prothese das Oberteil und das Unterteil in Schienen oder Befestigungsschalen zur Festlegung an der jeweiligen Gliedmaße oder Körperteil ausgebildet ist.

In einer Weiterbildung der Erfindung ist vorgesehen, dass der Aktuator bei der Verschwenkung um die Bewegungsachse eine Bewegung in einer Ebene, die durch den Rahmen festgelegt wird, durchführt. Die Bewegung des Aktuators ist dabei an die Bewegung des Rahmens gekoppelt.

Eine Weiterbildung der Erfindung sieht vor, dass zumindest ein Sensor zur Erfassung von Winkelstellungen, Wegen, Kräften, Momenten, Raumlagen und/oder Beschleunigungen an der Gelenkeinrichtung, dem Aktuator, dem Rahmen und/oder der Halterung angeordnet ist, um die Aktivitäten des Aktuators zu steuern. Dazu ist der

Sensor oder sind die Sensoren mit einer elektronischen Steuerungseinrichtung und gegebenenfalls einem Interface für einen Nutzer gekoppelt. Die Kopplung kann drahtgebunden oder drahtlos sein. In dem Interface kann eine drahtgebundene oder drahtlose Schnittstelle zur Einstellung der Steuerungsparameter des Aktuators angeordnet sein. Ebenfalls kann eine Energieversorgung zur Bereitstellung der benötigten elektrischen Energie für die Steuerung sowie für den Antrieb oder die Verstellung von Ventilen oder zur Erzeugung eines elektromagnetischen Feldes an der Gelenkeinrichtung, insbesondere an dem Rahmen vorgesehen sein.

Nachfolgend werden Ausführungsbeispiele der Erfindung anhand der beigefügten Figuren näher erläutert. Es zeigen:
- Figur 1 -: eine schematische Seitenansicht einer Gelenkeinrichtung in Gestalt einer Orthese;
- Figur 2 -: eine Frontalansicht mit einer lotrechten Ausrichtung von Oberteil zu Unterteil;
- Figur 3 -: eine lateral gekippte Anordnung des Aktuators;
- Figur 4 -: eine medial gekippte Anordnung des Aktuators;
- Figur 5 -: eine Draufsicht auf eine Gelenkeinrichtung in Gestalt eines Rahmens, der gelenkig mit einer Halterung verbunden ist; sowie

Figuren 6 bis 8 Darstellungen einer Variante der Erfindung..

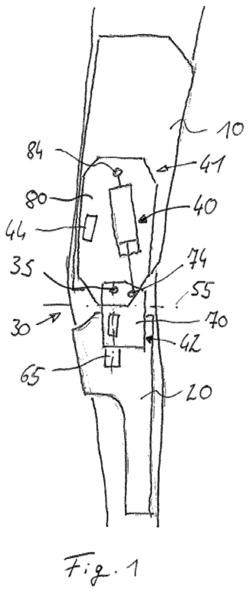

Figur 1 zeigt in einer Seitenansicht eine Gelenkeinrichtung an einer Orthese mit einem Oberteil 10, das als Schale oder Schiene zur Festlegung an einem Oberschenkel ausgebildet ist. An dem Oberteil 10 ist schwenkbar um eine Gelenkachse 35 ein Unterteil 20 angeordnet, das ebenfalls eine Schale oder als zumindest eine Schiene ausgebildet ist. Das Oberteil 10 und das Unterteil 20 sind über nicht dargestellte Befestigungselemente an der jeweiligen Gliedmaße angeordnet. Das Ausführungsbeispiel sieht eine Knieorthese vor, die an einem Bein eines Nutzers angelegt ist. Das Oberteil 10 ist damit als Oberschenkelschale oder als Oberschenkelschiene, gegebenenfalls als eine Kombination zweier Schienen, die medial und lateral an dem Oberschenkel angeordnet sind ausgebildet, wohingegen das Unterteil 20 als Unterschenkelschale oder als Unterschenkelschienen ausgebildet ist.

Die Gelenkachse 35 befindet sich in dem Bereich der natürlichen Schwenkachse des jeweiligen Gelenkes, im vorliegenden Ausführungsbeispiel des Kniegelenks. Da die natürliche Gelenkachse bei einem Kniegelenk aufgrund dessen komplexen Aufbaus veränderlich ist, befindet sich die Gelenkachse 35 in dem Bereich der natürlichen Gelenkachse oder an einer Kompromissgelenkachse.

Zwischen dem Oberteil 10 und dem Unterteil 20 ist ein Aktuator 40 angeordnet, über den eine Extensionsbewegung oder Flexionsbewegung um die Gelenkachse 35 beeinflusst werden kann. Der Aktuator 40 kann als passiver Aktuator ausgebildet sein, insbesondere als Hydraulikdämpfer, Pneumatikdämpfer oder als Bremseinrichtung, die elektrisch, pneumatisch, mechanisch oder magnetisch betrieben wird. Ebenfalls ist es möglich, den Aktuator 40 als Antrieb auszugestalten, der elektrisch, pneumatisch oder hydraulisch arbeitet. Dem jeweiligen Antrieb sind Energiebereitstellungseinrichtungen 44 zugeordnet, über die der jeweilige Antrieb mit der benötigten Energie versorgt wird. Als Energiebereitstellungseinrichtungen können Druckspeicher, elastische Elemente oder Batterien oder Akkumulatoren vorgesehen sein. Andere Energiespeicher sind ebenfalls möglich und vorgesehen. Ebenfalls ist es möglich, dass ein Antrieb 40 auch als Bremse eingesetzt wird. In dem dargestellten Ausführungsbeispiel ist der Aktuator 40 als Linearaktuator ausgebildet und weist ein Gehäuse auf, in dem ein Kolben oder ein anderer, linear verfahrbarer Kraftüberträger angeordnet ist. Im Falle eines Kolbens ist dieser mit einer Kolbenstange verbunden, deren Ende an einer distalen Lagerstelle 74 an einer Halterung 70 gelagert ist. Das Gehäuse ist an einer oberen Lagerstelle 84 an einem Rahmen 80 angeordnet. Über den Rahmen 80 und die Halterung 70 wird eine obere Befestigungsstelle 41 und eine untere Befestigungsstelle 42 ausgebildet, so dass der Aktuator 40 über den Rahmen 80 und die Halterung 70 an dem Oberteil 10 und dem Unterteil 20 an einer Oberteilbefestigungsstelle 41 und einer Unterteilbefestigungsstelle 42 gelagert ist. Es ist auch möglich, die obere Lagerstelle 84 unmittelbar an dem Oberteil 10 vorzusehen. Ebenso ist es möglich, eine umgekehrte Anordnung von Rahmen und Halterung vorzusehen oder den Aktuator 40 mit dem Gehäuse an der Halterung 70 an dem Unterteil 20 festzulegen.

In dem dargestellten Ausführungsbeispiel wird über den Rahmen 80 und die Halterung 70 das Gelenk 30 ausgebildet. Innerhalb der Halterung 70 und zwischen der Lagerstelle 74 an der Halterung 70 und der Unterteilbefestigungsstelle 42 sind zwei Gelenke angeordnet, von denen nur deren Schwenkachsen 55, 65 aufgrund der schematischen Darstellung gezeigt sind. Um die Schwenkachsen 55, 65 kann die Halterung 70 und damit auch der Aktuator 40 relativ zu dem Unterteil 20 verschwenken, wobei der Verschwenkbereich um die Schwenkachsen 55, 65 über Anschläge begrenzt ist. In dem dargestellten Ausführungsbeispiel stehen alle drei Achsen, die Gelenkachse 35 sowie die Schwenkachsen 55, 65 senkrecht aufeinander. Dies muss aber nicht unbedingt der Fall sein. Bevorzugt schneiden sich alle drei Achsen 35, 55, 65 in einem Punkt, um die mediale und laterale Verschwenkbarkeit sowie eine Rotation um die Längserstreckung des Unterteils 20 beziehungsweise der Gliedmaße ausführen zu können, ohne die Verschwenkbewegung um die Gelenkachse 35 zu beeinträchtigen.

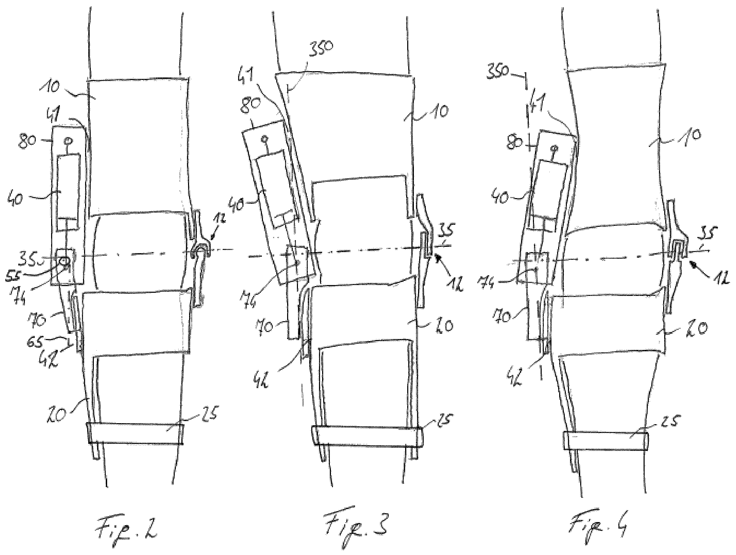

In der Figur 2 ist eine Frontalansicht der Ausgestaltung gemäß Figur 1 gezeigt. In der Figur 2 sind die Oberteilbefestigungsstelle 41 und die Unterteilbefestigungsstelle 42 besser zu erkennen. Die Gelenkachse 35 erstreckt sich im Wesentlichen horizontal durch das natürliche Gelenk. Die Orthese weist auf der dem Aktuator 40 gegenüberliegenden Seite ein Mitläufergelenk 12 auf, um einer verbesserte Führung des Oberteils 10 relativ zum Unterteil 20 zu ermöglichen. In der Figur 2 sind ebenfalls Befestigungsmittel 25 in Gestalt eines Gurtes zu erkennen, über den das Unterteil 20 an dem Unterschenkel festlegbar ist. Korrespondierende Befestigungsmittel sind an dem Oberteil 10 vorgesehen. In Ausgestaltung gemäß Figur 2 ist das Bein gerade, das Oberteil 10 und das Unterteil 20 sind entsprechend gerade zueinander ausgerichtet, die jeweilige Längserstreckung von Oberteil 10 und Unterteil 20 verläuft in einer gemeinsamen Ebene oder in zueinander parallelen oder im Wesentlichen parallelen Ebenen. Die äußeren Oberflächen des Oberteils 10 und des Unterteils 20 liegen im Wesentlichen in einer Ebene senkrecht zu der Gelenkachse 35, so dass sowohl der Aktuator 40 als auch der Rahmen 30 und die Halterung 70 sehr eng anliegend an dem Körper beziehungsweise an dem Oberteil 10 und dem Unterteil 20 festlegbar sind. Eine Verdrehbarkeit oder Verschwenkbarkeit um eine der Schwenkachsen 55, 65 ist nicht oder nur in einem geringen Maße notwendig, um Fehlausrichtungen zu kompensieren.

In der Figur 3 ist eine mögliche Variante einer Oberteilausgestaltung dargestellt. Das Oberteil 10 ragt seitlich über eine Schwenkebene 350, die senkrecht zu der Gelenkachse 35 orientiert ist, hinaus, der Aktuator 40 mit dem Rahmen 80 ist aus der Schwenkebene 350 um circa 35 Grad verkippt. Um diese Verkippung auszugleichen, ist in der Halterung 70 ein Gelenk vorgesehen, das eine Verschwenkung um die Schwenkachse 55 ermöglicht. Durch die Verschwenkung des Aktuators 40 aus der Schwenkebene 350 heraus kommt es bei einer Aktuierung zu einem Drehmoment, das eine Verschwenkung um die senkrechte Schwenkachse 65 bewirkt. Durch die Ausführung eines um die Schwenkachse verdrehbaren Gelenks 60 ist es möglich, dass eine Rotation des Aktuators 40 vermieden wird, wenn das Gelenk 30 flektiert oder extendiert wird.

Eine korrespondierende Ausgestaltung ist in der Figur 4 gezeigt, bei der statt einer lateralen Auslenkung des Aktuators 40 eine mediale Auslenkung erfolgt.

In den Figuren 2 bis 4 sind jeweils unterschiedliche Mitläufergelenke 12 dargestellt, in der Figur 2 ist es ein Kugelgelenk, in der Figur 3 ein Scharniergelenk und in der Figur 4 ein Kardangelenk.

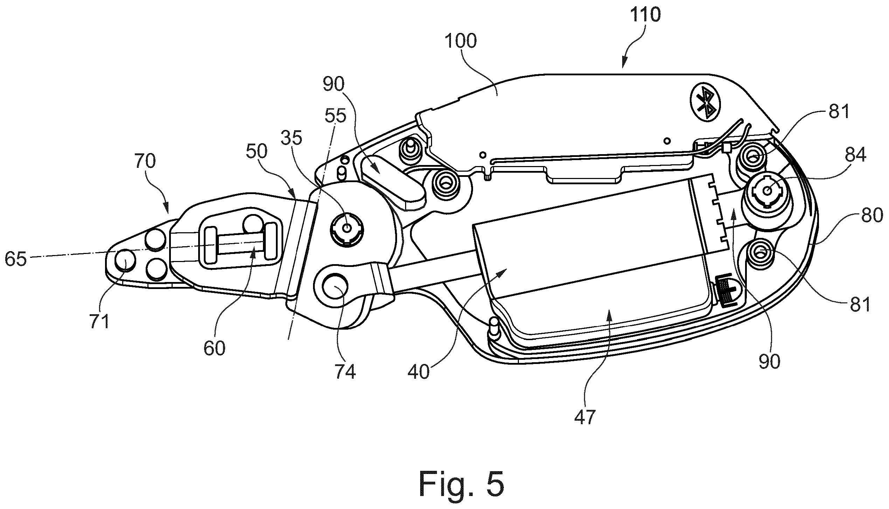

Figur 5 zeigt in einer Detailansicht den Aktuator 40 als Linearaktuator in Gestalt eines Hydraulikdämpfers mit einer Ventileinheit 47, die über eine Steuerelektronik 100 angesteuert werden kann. Die Elektronikeinheit 100 ist mit Sensoren 90 gekoppelt, die im dargestellten Ausführungsbeispiel Kräfte und Winkelstellungen erfassen. Dargestellt ist ein Winkelsensor 90 in der Nähe der Gelenkachse 35 sowie ein Kraftsensor 90 an einer oberen Lagerstelle 84 des Aktuators 40. Die Lagerstelle 84 ist an einem Rahmen 80 ausgebildet, auf dem sowohl der Aktuator 40 als auch die Steuereinheit 100 sowie ein Nutzer-Interface 110 angeordnet ist. An der Steuereinheit 100 kann eine zusätzliche Sensoreinrichtung angeordnet sein, die die Raumlage erfasst. Ein sogenannter Raumlagesensor liefert Daten über die Stellung des Rahmens 80 oder des Aktuators 40 im Raum, insbesondere über die Orientierung relativ zu der Schwerkraftrichtung.

An dem Rahmen 80, der beispielsweise aus einem formstabilen Kunststoff, ggf. faserverstärkten Kunststoff oder einem Metall ausgebildet sein kann, sind mehrere Befestigungseinrichtungen 81 in Gestalt von Zapfen oder Durchgangsbohrungen vorhanden, über die der Rahmen 80 an dem nicht dargestellten Oberteil 10 reversibel festlegbar ist. Ebenfalls können an dem Rahmen 80 weitere Komponenten, insbesondere Energiespeicher angeordnet sein. An dem Rahmen 80 können verschiedene Lagerstellen 84 vorbereitet sein, um unterschiedliche Aktuatoren oder Komponenten einfach an dem Rahmen 80 festlegen zu können.

Schwenkbar um die Gelenkachse 35 ist an dem Rahmen 80 eine Halterung 70 angeordnet, die zusammen mit dem Rahmen 80 die Gelenkeinrichtung ausbildet. Die Halterung 70 wird über Befestigungseinrichtungen 71 an einer Halteplatte 70a an dem nicht dargestellten Unterteil 20 vorzugsweise drehfest festgelegt. Die Befestigungseinrichtungen 71 können als Nieten oder Schrauben oder ähnliche, bevorzugt formschlüssig wirkende Befestigungselemente ausgebildet sein. Über die Befestigungseinrichtungen 71 kann die Halterung 70 über die Halteplatte 70a reversibel an einem Unterteil 20 festgelegt werden.

Innerhalb der Halterung 70 sind zwei Gelenke 50, 60 ausgebildet, die eine Verschwenkung der Lagerstelle 74 relativ zu der Halteplatte 70a ermöglichen. Ein erstes Gelenk 50 ermöglicht eine Verschwenkung um eine erste Schwenkachse 55. Diese erste Schwenkachse 55 liegt in einer Ebene orthogonal zu der Gelenkachse 35 und ermöglicht eine Verkippung in einer angenäherten Medial-Lateral-Richtung, wenn der Rahmen 80 und die Halterung 70 seitlich an der Außenseite einer Orthese angeordnet sind. In derselben Ebene liegt die zweite Schwenkachse 65, die durch das zweite Gelenk 60 ausgebildet wird. Die Schwenkachse 65 erstreckt sich im Wesentlichen in Längserstreckung der Gelenkeinrichtung und geht bevorzugt durch die Gelenkachse 35 hindurch. Ebenso ist es bevorzugt, wenn die erste Schwenkachse 55 durch die Gelenkachse 35 hindurchgeht. Die beiden Schwenkachsen 55, 65 können, müssen aber nicht in einem rechten Winkel zueinander stehen, im dargestellten Ausführungsbeispiel stehen die beiden Schwenkachsen 55, 65 nicht senkrecht aufeinander. Die Gelenke 50, 60 sind an der Halterung 70 ausgebildet, die verdrehbar mit der Halteplatte 70a verbunden ist. Diese ist Teil der Halterung 70 und mit dem Unterteil 20 drehfest verbunden ist.

An der Halterung 70 ist darüber hinaus beabstandet zu der Gelenkachse 35 eine distale Lagerstelle 74 für die Lagerung der Kolbenstange an der Halterung 70 ausgebildet. Die Lagerstelle 74 ermöglicht eine Verschwenkung um eine Achse im Wesentlichen parallel zu der Gelenkachse 35.

Die Befestigung des Aktuators 40 an dem Oberteil 10 und dem Unterteil 20 erfolgt über den Rahmen 80 einerseits und die Halterung 70 andererseits. Der Rahmen 80 und die Halteplatte 70a bilden über ihre jeweiligen Befestigungseinrichtungen 71, 81 die Oberteilbefestigungsstelle 41 und die Unterteilbefestigungsstelle 42 aus. Der Linearaktuator 40 ist über die Lagerstelle 84 verdrehbar an dem Rahmen 80 festgelegt. Ebenso sind die Halteplatte 70a und der Rahmen 80 drehfest an dem Unterteil 20 bzw. Oberteil 10 festgelegt, so dass bei einer Verschwenkung um die Gelenkachse 35 die Kolbenstange in das Gehäuse des Linearaktuators 40 ein- bzw. ausfährt.

Unter Bezugnahme auf die Figuren 2 bis 4 wird deutlich, dass vor allem in der Orthetik, allerdings auch in der Prothetik, die Situation auftreten kann, dass die verwendeten Komponenten möglichst nahe an den noch erhaltenen Körperteilen angebracht werden sollen, um ein möglichst geringes, zusätzliches Volumen an dem Körper zu erzielen. Über den Aktuator 40 kann eine gezielte Unterstützung eines Momentes in Flexions- und Extensionsrichtung in verschiedenen Phasen der Bewegung, beispielsweise des Gehens, erfolgen, jeweils dann, wenn eine Unterstützung oder Führung erforderlich ist. Dazu wird der Aktuator 40 so an der Gelenkeinrichtung angelenkt, dass die Linearbewegung des Aktuators ein unterstützendes oder der Bewegung widerstehendes Moment bewirkt. Während es früher notwendig war, über Distanzstücke eine stets in der Schwenkebene 350 liegende Orientierung des Aktuators 40 zu gewährleisten, ist es mit der erfindungsgemäßen Gelenkeinrichtung möglich, dass die Anlenkung des Aktuators 40 und die übrige mechanische Struktur zur Lastübertragung anpassbar an die Körperkontur oder an die jeweils unterschiedlichen Ausgestaltungen der Orthese oder Prothese möglich ist, ohne dass eine manuelle Bearbeitung wie Verformung oder das Anordnen von Zwischenstücken erforderlich wird. Dabei erfolgt die Bewegung des Linearaktuators 40 in einer Ebene, die in einer von der Schwenkebene 350, die lotrecht zu der Gelenkachse 35 orientiert ist, abweicht. Die Anordnung in nur einer Ebene ermöglicht es, das benötigte Bauvolumen gering zu halten. Mit solchen Gelenkeinrichtungen können kompakte, mechatronische Orthesen oder Prothesen gebaut werden. In der Prothetik ist beispielsweise ein Einsatz bei Patienten mit einem sehr langen Stumpf oder bei kleinen Patienten möglich. Ein einfaches Prothesengelenk mit einem Scharniergelenk kann durch eine seitliche Montage einer erfindungsgemäßen Gelenkeinrichtung mit einer verbesserten Funktionalität ausgestattet werden. Gleiches gilt für Prothesen des Sprunggelenkes oder der Hüfte, grundsätzlich auch für Prothesen der oberen Extremität. Über die Gelenkeinrichtung, insbesondere bei einer modularen Ausgestaltung mit einem Rahmen 80 und einer Halteplatte 70a, können Testprothesen oder Testorthesen leicht hergestellt werden. Darüber hinaus können unterschiedliche Aktuatoren wie Dämpfer oder Antriebe einfach an bevorzugten standardisierten Befestigungspunkten an dem Oberteil und dem Unterteil festgelegt werden, so dass eine große Variation an Komponenten an dem jeweiligen Patienten getestet werden kann.

Mit der erfindungsgemäßen Gelenkeinrichtung kann die Positionierung des Aktuators 40 und die mechanische Struktur zur Lastübertragung in Gestalt des Oberteils und des Unterteils in seiner Winkelausrichtung in allen drei Raumrichtungen variiert werden, insbesondere in der Frontalebene um eine anterior-posterior-Achse, ohne dass manuelle Anpassungen oder Distanzelemente notwendig sind. Wenn die an die Körperkontur des Anwenders optimierte Winkelposition des Aktuators gefunden ist, kann diese optimale Winkelposition durch ein externes Mitläufergelenk 12 fixiert werden. Das Mitläufergelenk 12 ist auf der kontralateralen Seite, insbesondere auf der medialen Seite, angeordnet, da die Aktuatoreneinheit in der Regel lateral an einem Patienten angeordnet ist. Die Rotationsachse einer Orthese, beispielsweise die Gelenkachse 35, wird bevorzugt durch zwei Punkte, einmal der Gelenkeinrichtung 30 an der Seite des Aktuators 40 und einmal an der kontralateralen Seite an einem Mitläufergelenk 12, realisiert.

Durch die erfindungsgemäße Ausgestaltung kann sich der Aktuator auf einer Bahn bewegen, die von der Schwenkebene 350, die durch eine auf die Kompromissdrehachse des Gelenkes lotrechten Ebene definiert wird, abweicht. Durch die zusätzlichen Gelenke 50, 60 wird eine Rotation des Aktuators 40 vermieden und das überstrichene Volumen bei der Bewegung miniert, wodurch das benötigte Bauvolumen geringer als bei bisher bekannten Ausführungen ist. Die Ausrichtung der mechanischen Komponenten auf dem Oberteil 10 und dem Unterteil 20 wird im Zuge des Aufbaus einer Orthese oder Prothese definiert. Im Falle einer Orthese wird über das mechanische Mitläufergelenk 12 die Orthese stabilisiert und in ihren Freiheitsgraden ausreichend definiert.

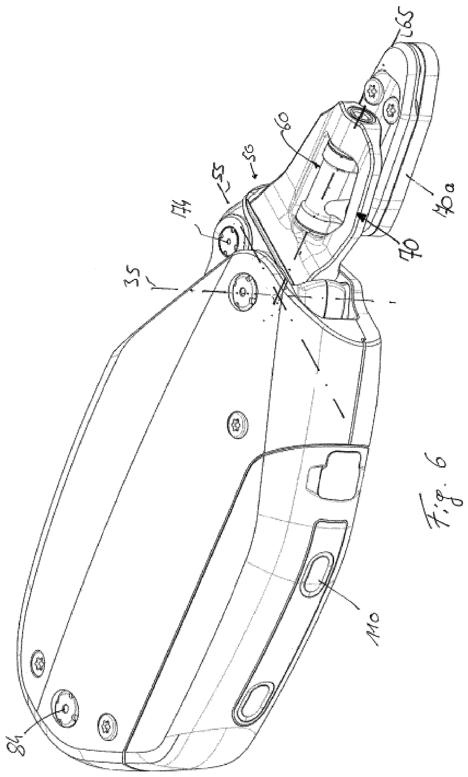

In den Figuren 6 bis 8 ist eine weitere Variante der Erfindung dargestellt, bei der der Aktuator 40 an dem Rahmen 80 hinter einer Verkleidung verborgen ist. Von außen zugänglich ist, wie in der Figur 6 zu sehen, die Schnittstelle 110 als Interface zwischen der an dem Rahmen befestigten Steuerungseinrichtung 100 und dem Nutzer oder einer externen Computereinrichtung. Die obere, proximale Lagerstelle 84 ist ebenso zu erkennen wie die untere, distale Lagerstelle 74. Die Halterung 70 weist zwei Gelenke 50, 60 auf, wobei die Schwenkachsen 55, 65 der beiden Gelenke 50, 60 sich in der Schwenkebene 350 orthogonal zu der Gelenkachse 35 befinden. Die Gelenkachse 35 wird durch eine kardangelenkartige Verbindung zwischen der Halterung 70 und dem Rahmen 80 ausgebildet und über das Mitläufergelenk 12 festgelegt. Beide Schwenkachsen 55, 65 schneiden in sich in der Gelenkachse 35.

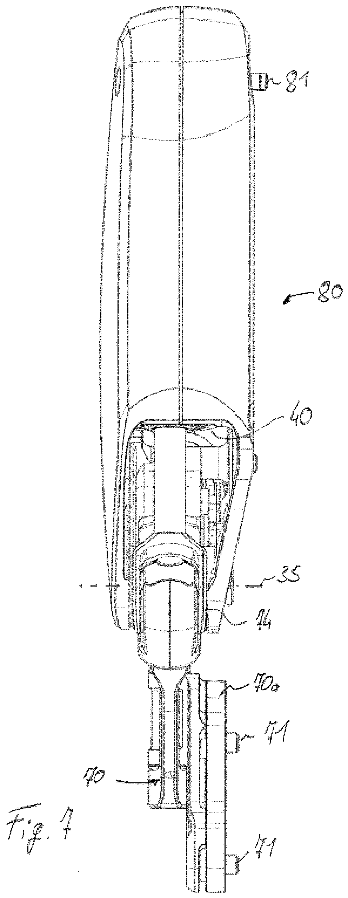

Figur 7 zeigt eine Hintenansicht der Gelenkeinrichtung mit dem Rahmen 80 und der Halterung 70 und den jeweils seitlichen, in Medialrichtung herausstehenden Befestigungseinrichtungen 71, 81, beispielsweise als Bolzen oder Schraubaufnahmen. Die Gelenkachse 35 ist ebenso zu erkennen wie der Linearaktuator 40 innerhalb des Gehäuses an dem Rahmen 80 sowie die distale Lagerstelle 74.

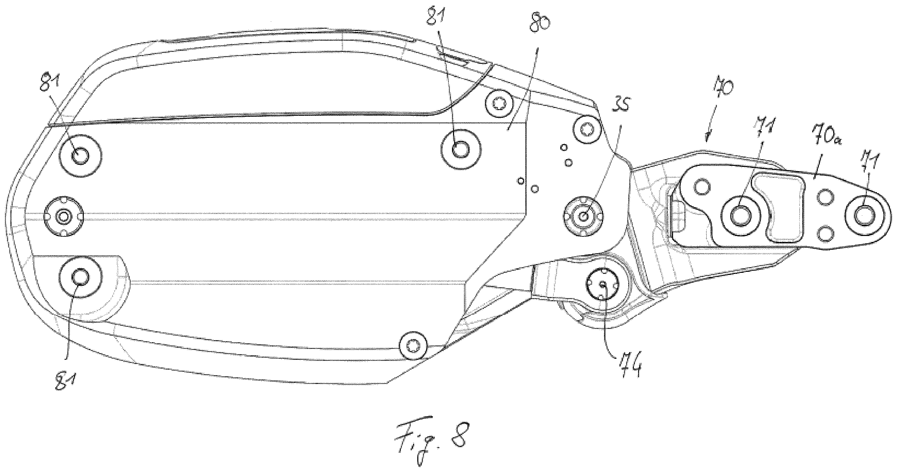

Figur 8 zeigt eine Seitenansicht, bei der auf den Rahmen 80 aus Medialrichtung, also aus Richtung der Orthese, draufgeschaut wird. Die Gelenkachse 35 erstreckt sich orthogonal aus der Blattebene heraus, die Blattebene bildet dementsprechend die Schwenkebene 350. Die distale Lagerstelle 74 des Linearaktuators ist beabstandet zu der Gelenkachse 35 angeordnet, um ein Moment um die Gelenkachse 35 herum aufzubauen bzw. Kräfte aufnehmen zu können. An der der Orthese zugewandten Seite des Rahmens 80 sind insgesamt drei Befestigungseinrichtungen 81 ausgebildet, über die es möglich ist, den Rahmen 80 stabil und drehfest an dem Oberteil zu befestigen. An der Halteplatte 70a sind zwei Befestigungseinrichtungen 71 angeordnet, über die die Halteplatte 70a drehfest und ortsstabil an dem nicht dargestellten Unterteil festlegbar ist. Über die Befestigungseinrichtungen 71, 81 kann eine reversible Befestigung der Gelenkeinrichtung, die in diesem Fall über den Rahmen 80 und die Halterung 70 ausgebildet wird, erfolgen.

Abweichend von einer Anordnung über einen Rahmen 80 kann der Aktuator auch direkt an einem Oberteil oder einem Unterteil befestigt sein.

## Patentansprüche

1. Gelenkeinrichtung einer Orthese oder Prothese oder für eine Orthese oder Prothese mit
- einem Oberteil (10),
- einem Unterteil (20),
- einem Gelenk (30), das eine Gelenkachse (35) aufweist, um die das Oberteil (10) relativ zu dem Unterteil (20) verschwenkbar gelagert ist, und
- einem Aktuator (40), der ausgebildet ist, eine Verschwenkung des Oberteils (10) relativ zu dem Unterteil (20) zu beeinflussen, wobei
- der Aktuator (40) an dem Oberteil (10) an einer Oberteilbefestigungsstelle (41) und an dem Unterteil (20) an einer Unterteilbefestigungsstelle (42) gelagert ist, wobei zwischen der Oberteilbefestigungsstelle (41) und der Unterteilbefestigungsstelle (42) zumindest zwei Gelenke (50, 60) angeordnet sind, die Gelenke (50, 60) eine Verschwenkung des Aktuators (40) zu der Oberteilbefestigungsstelle (41) oder der Unterteilbefestigungsstelle (42) ermöglichen und jeweils zumindest eine Schwenkachse (55, 65) ausbilden, von denen zumindest eine nicht parallel zu der Gelenkachse (35) orientiert ist, **dadurch gekennzeichnet, dass** die Gelenkeinrichtung eine Halterung (70) umfasst, wobei der Aktuator (40) an dieser Halterung (70) befestigt ist, die zwischen der Gelenkachse (35) und der Oberteilbefestigungsstelle (41) oder der Unterteilbefestigungsstelle (42) angeordnet ist, wobei in der Halterung (70) zumindest eine der Schwenkachsen (55, 65) ausgebildet ist.

2. Gelenkeinrichtung nach Anspruch 1, **dadurch gekennzeichnet, dass** eine erste Schwenkachse (55) orthogonal zu der Gelenkachse (35) orientiert ist.

3. Gelenkeinrichtung nach Anspruch 1 oder 2, **dadurch gekennzeichnet, dass** die erste Schwenkachse (55) orthogonal zu einer zweiten Schwenkachse (65) orientiert ist.

4. Gelenkeinrichtung nach einem der voranstehenden Ansprüche, **dadurch gekennzeichnet, dass** beide Schwenkachsen (55, 65) nicht parallel zueinander und zu der Gelenkachse (35) orientiert sind.

5. Gelenkeinrichtung nach einem der voranstehenden Ansprüche, **dadurch gekennzeichnet, dass** die Schwenkachsen (55, 65) in einer gemeinsamen Ebene liegen.

6. Gelenkeinrichtung nach einem der voranstehenden Ansprüche, **dadurch gekennzeichnet, dass** der Aktuator (40) als Linearaktuator ausgebildet ist.

7. Gelenkeinrichtung nach einem der voranstehenden Ansprüche, **dadurch gekennzeichnet, dass** der Aktuator (40) als elektrischer, pneumatischer oder hydraulischer Antrieb oder als hydraulischer oder pneumatischer Dämpfer oder elektrische, pneumatische, mechanische oder magnetische Bremse ausgebildet ist.

8. Gelenkeinrichtung nach einem der voranstehenden Ansprüche, **dadurch gekennzeichnet, dass** die Schwenkachsen (55, 65) und die Gelenkachse (35) sich in einem gemeinsamen Punkt schneiden.

9. Gelenkeinrichtung nach einem der voranstehenden Ansprüche, **dadurch gekennzeichnet, dass** zumindest eine der Schwenkachsen (55, 65) in einer Schwenkebene (350) liegt, die senkrecht zu der Gelenkachse (35) orientiert ist.

10. Gelenkeinrichtung nach Anspruch 1, **dadurch gekennzeichnet, dass** die Halterung (70) eine Halteplatte (70a) aufweist, die drehfest an dem Oberteil (10) oder dem Unterteil (20) festgelegt ist.

11. Gelenkeinrichtung nach einem der voranstehenden Ansprüche, **dadurch gekennzeichnet, dass** die Gelenkachse (35) zwischen dem Oberteil (10) und der Halterung (70) oder zwischen dem Unterteil (20) und der Halterung (70) ausgebildet ist.

12. Gelenkeinrichtung nach einem der voranstehenden Ansprüche, **dadurch gekennzeichnet, dass** der Aktuator (40) als Linearaktuator ausgebildet ist und an einer Lagerstelle (74) beabstandet zu der Gelenkachse (35) an der Halterung (70) gelagert ist.

13. Gelenkeinrichtung nach einem der voranstehenden Ansprüche, **dadurch gekennzeichnet, dass** der Aktuator (40) an einem Rahmen (80) festgelegt ist, der Befestigungseinrichtungen (81) zur reversiblen Anordnung an dem Oberteil (10) oder dem Unterteil (20) aufweist.

14. Gelenkeinrichtung nach Anspruch 13, **dadurch gekennzeichnet, dass** die Halterung (70) schwenkbar an dem Rahmen (80) befestigt ist und die Gelenkachse (35) und/oder die Schwenkachse (55) zwischen dem Rahmen (80) und der Halterung (70) ausgebildet ist.

15. Gelenkeinrichtung nach Ansprüchen 13 oder 14, **dadurch gekennzeichnet** das der Aktuator (40) bei der Verschwenkung um die Bewegungsachse (35) eine Bewegung in einer Ebene, die durch den Rahmen (80) festgelegt wird, durchführt.

16. Gelenkeinrichtung nach einem der voranstehenden Ansprüche, **dadurch gekennzeichnet, dass** zumindest ein Sensor (90) zur Erfassung von Winkelstellungen, Wegen, Kräften, Momenten, Raumlagen und/oder Beschleunigungen an der Gelenkeinrichtung, dem Aktuator (40), dem Rahmen (80) , und/oder der Halterung (70) angeordnet ist.

## Claims

1. A joint device of an orthosis or prosthesis or for an orthosis or prosthesis with
- an upper part (10),
- a lower part (20),
- a joint (30) that comprises a joint axis (35), about which the upper part (10) is mounted such that it can be swivelled relative to the lower part (20), and
- an actuator (40), which is designed to influence a swivelling of the upper part (10) relative to the lower part (20), wherein
- the actuator (40) is mounted at an upper part fixing point (41) on the upper part (10) and at a lower part fixing point (42) on the lower part (20), wherein at least two joints (50, 60) are arranged between the upper part fixing point (41) and the lower part fixing point (42); the joints (50, 60) enable a swivelling of the actuator (40) relative to the upper part fixing point (41) or the lower part fixing point (42), and they each form at least one joint axis (55, 65), at least one of which is not oriented parallel to the joint axis (35), **characterized in that** the joint device comprises a bracket (70), wherein the actuator (40) is fixed to this bracket (70), which is arranged between the joint axis (35) and the upper part fixing point (41) or the lower part fixing point (42), wherein at least one of the swivel axis (55, 65) is configured in the bracket (70).

2. The joint device according to claim 1, **characterized in that** a first swivel axis (55) is oriented orthogonally to the joint axis (35).

3. The joint device according to claim 1 or 2, **characterized in that** the first swivel axis (55) is oriented orthogonally to a second swivel axis (65).

4. The joint device according to one of the above claims, **characterized in that** both swivel axes (55, 65) are oriented neither parallel to each another nor to the joint axis (35).

5. The joint device according to one of the above claims, **characterized in that** the swivel axes (55, 65) lie in a common plane.

6. The joint device according to one of the above claims, **characterized in that** the actuator (40) is designed as a linear actuator.

7. The joint device according to one of the above claims, **characterized in that** the actuator (40) is designed as an electrical, pneumatic or hydraulic drive, or a hydraulic or pneumatic damper, or an electrical, pneumatic, mechanical or magnetic brake.

8. The joint device according to one of the above claims, **characterized in that** the swivel axes (55, 65) and the joint axis (35) intersect at a common point.

9. The joint device according to one of the above claims, **characterized in that** at least one of the swivel axes (55, 65) lies in a swivel plane (350), which is oriented perpendicular to the joint axis (35).

10. The joint device according to claim 1, **characterized in that** the bracket (70) comprises a mounting plate (70a), which is attached to the upper part (10) or the lower part (20) such that it is torque-proof.

11. The joint device according to one of the above claims, **characterized in that** the joint axis (35) is configured between the upper part (10) and the bracket (70) or between the lower part (20) and the bracket (70).

12. The joint device according to one of the above claims, **characterized in that** the actuator (40) is designed as a linear actuator and is mounted on the bracket (70) at a bearing point (74) at a distance from the joint axis (35).

13. The joint device according to one of the above claims, **characterized in that** the actuator (40) is attached to a frame (80), which comprises fixing devices (81) for the reversible arrangement on the upper part (10) or the lower part (20).

14. The joint device according to claim 13, **characterized in that** the bracket (70) is fixed to the frame (80) such that it can be swivelled and the joint axis (35) and/or the swivel axis (55) is configured between the frame (80) and the bracket (70).

15. The joint device according to claims 13 or 14, **characterized in that**, when the swivelling about the axis of movement (35) occurs, the actuator (40) conducts a movement in the plane that is defined by the frame (80).

16. The joint device according to one of the above claims, **characterized in that** at least one sensor (90) is arranged for recording angular positions, paths, forces, torques, spatial positions and/or accelerations on the joint device, the actuator (40), the frame (80) and/or the bracket (70).

## Revendications

1. Dispositif d'articulation d'une orthèse ou d'une prothèse ou pour une orthèse ou une prothèse, comprenant
- une partie supérieure (10),
- une partie inférieure (20),
- une articulation (30) présentant un axe d'articulation (35) autour duquel la partie supérieure (10) peut pivoter par rapport à la partie inférieure (20), et
- un actionneur (40) conçu pour influencer un pivotement de la partie supérieure (10) par rapport à la partie inférieure (20), dans lequel
- l'actionneur (40) est monté sur la partie supérieure (10) en un point de fixation de partie supérieure (41) et sur la partie inférieure (20) en un point de fixation de partie inférieure (42), au moins deux articulations (50, 60) étant disposées entre le point de fixation de partie supérieure (41) et le point de fixation de partie inférieure (42), les articulations (50, 60) permettant un pivotement de l'actionneur (40) par rapport au point de fixation de partie supérieure (41) et au point de fixation de partie inférieure (42) et formant chacune au moins un axe de pivotement (55, 65) dont l'un au moins est orienté de façon non parallèle à l'axe d'articulation (35),
**caractérisé en ce que** le dispositif d'articulation comprend un support (70), l'actionneur (40) étant fixé à ce support (70) qui est disposé entre l'axe d'articulation (35) et le point de fixation de partie supérieure (41) ou le point de fixation de partie inférieure (42), l'un au moins des axes de pivotement (55, 65) étant formé dans le support (70).

2. Dispositif d'articulation selon la revendication 1,
**caractérisé en ce qu'**un premier axe de pivotement (55) est orienté orthogonalement à l'axe d'articulation (35).

3. Dispositif d'articulation selon la revendication 1 ou 2,
**caractérisé en ce que** le premier axe de pivotement (55) est orienté orthogonalement à un deuxième axe de pivotement (65).

4. Dispositif d'articulation selon l'une des revendications précédentes,
**caractérisé en ce que** les deux axes de pivotement (55, 65) sont orientés de façon non parallèle l'un à l'autre et à l'axe d'articulation (35).

5. Dispositif d'articulation selon l'une des revendications précédentes,
**caractérisé en ce que** les axes de pivotement (55, 65) sont situés dans un plan commun.

6. Dispositif d'articulation selon l'une des revendications précédentes,
**caractérisé en ce que** l'actionneur (40) est conçu comme un actionneur linéaire.

7. Dispositif d'articulation selon l'une des revendications précédentes,
**caractérisé en ce que** l'actionneur (40) est conçu comme un entraînement électrique, pneumatique ou hydraulique ou comme un amortisseur hydraulique ou pneumatique ou comme un frein électrique, pneumatique, mécanique ou magnétique.

8. Dispositif d'articulation selon l'une des revendications précédentes,
**caractérisé en ce que** les axes de pivotement (55, 65) et l'axe d'articulation (35) se coupent en un point commun.

9. Dispositif d'articulation selon l'une des revendications précédentes,
**caractérisé en ce que** l'un au moins des axes de pivotement (55, 65) est situé dans un plan de pivotement (350) orienté perpendiculairement à l'axe d'articulation (35).

10. Dispositif d'articulation selon la revendication 1,
**caractérisé en ce que** le support (70) comporte une plaque de maintien (70a) qui est fixée solidairement en rotation sur la partie supérieure (10) ou sur la partie inférieure (20).

11. Dispositif d'articulation selon l'une des revendications précédentes,
**caractérisé en ce que** l'axe d'articulation (35) est formé entre la partie supérieure (10) et le support (70) ou entre la partie inférieure (20) et le support (70).

12. Dispositif d'articulation selon l'une des revendications précédentes,
**caractérisé en ce que** l'actionneur (40) est conçu comme un actionneur linéaire et est monté sur le support (70) au niveau d'un point d'appui (74) situé à distance de l'axe d'articulation (35).

13. Dispositif d'articulation selon l'une des revendications précédentes,
**caractérisé en ce que** l'actionneur (40) est fixé sur un cadre (80) qui comprend des moyens de fixation (81) permettant un agencement réversible sur la partie supérieure (10) ou sur la partie inférieure (20).

14. Dispositif d'articulation selon la revendication 13,
**caractérisé en ce que** le support (70) est fixé de manière pivotante sur le cadre (80), et l'axe d'articulation (35) et/ou l'axe de pivotement (55) est formé entre le cadre (80) et le support (70).

15. Dispositif d'articulation selon les revendications 13 ou 14,
**caractérisé en ce que** l'actionneur (40) effectue, lors du pivotement autour de l'axe de mouvement (35), un mouvement dans un plan qui est défini par le cadre (80).

16. Dispositif d'articulation selon l'une des revendications précédentes,
**caractérisé en ce qu'**au moins un capteur (90) destiné à détecter des positions angulaires, des courses, des forces, des moments, des positions dans l'espace et/ou des accélérations est agencé sur le dispositif d'articulation, sur l'actionneur (40), sur le cadre (80) et/ou sur le support (70).
